# EUROPEAN PATENT APPLICATION

(11) **EP 0 524 663 A1**
(43) Date of publication of application: **27.01.1993**
(21) Application number: 92116475.2
(22) Date of filing: 27.01.1989
(51) Int. Cl.: B01J 20/06, C07K 17/14, C12N 11/14

(54) **High stability porous zirconium oxide spherules and support comprising same**

(30) Priority: 03.02.1988 US 151819
(62) Divisional of application: 89300824.3
(71) Applicant: THE REGENTS OF THE UNIVERSITY OF MINNESOTA, Minneapolis, Minnesota 55455 (US)
(72) Inventor: Carr, Peter W., Minneapolis, Minnesota 55414 (US); Rigney, Martin P., St. Paul, Minnesota 55104 (US); Funkenbusch, Eric F., White Bear Lake, Minnesota 55110 (US); Coleman, Patrick L., Minneapolis, Minnesota 55410 (US); Hanggi, Douglas A., St. Paul, Minnesota 55119 (US)
(74) Representative: Baillie, Iain Cameron

(57) **Abstract**

The present invention provides stable porous ZrO₂ spherules wherein said spherules have a pore diameter from 0.05-50 nm and a particle diameter range of 20-500 µm (microns), and are stable in aqueous media of pHs 1-14 and which comprise an immobilized protein.

## Description

Currently-known inorganic chromatography supports comprising particulate silica (SiO₂) or alumina (Al₂O₃) are stable over pH ranges of about 1-8 and 3-12, respectively. The solubilization of SiO₂ and Al₂O₃ at pHs outside of these ranges results in deterioration of these supports and contamination of the resultant chromatographed and separated products with silicon- or aluminum-containing species. Methods of improving the alkaline stability of particulate SiO₂ by cladding the surface with a more base-stable metal oxide such as zirconium oxide (ZrO₂) have been disclosed in U.S. Patent Nos. 4,648,975 and 4,600,646. This cladding is disclosed to increase the upper pH limit at which these supports, also referred to as packings, can be used to 11 and 9.5, respectively. However, these packings still lack adequate stability to allow them to be sterilized and cleaned in, for example, 0.1 N aqueous sodium hydroxide (NaOH, pH=13).

Use of porous spherical ZrO₂ particles on a thin layer chromatography plate has been disclosed in U.S. Patent No. 4,138,336, a process for the preparation of porous ZrO₂ microspheres is taught in U.S. Patent No. 4,010,242, and chromatographic use of these particles is taught in U.S. Patent No. 3,782,075. The microspheres are prepared by a process in which colloidal metal oxide particles are mixed with a polymerizable organic material and coacervated into spherical particles by initiating polymerization of the organic material. This is a time consuming, batch process which requires the addition of organic material which is pyrolized and hence lost.

U.S. Patent No. 3,862,908 discloses microspheres of urania and other metal oxides, however, these particles are fired to near full density, have reduced surface areas and therefore, would not be attractive for chromatographic uses.

U.S. Patent No. 3,892,580 discloses a process for preparing porous bodies of ZrO₂. This process requires the use of a binder to react with the oxide particles during preparation. This binder is subsequently decomposed by pyrolysis and therefore lost. The bodies produced by this process are not spherical, would pack unevenly, may cause increased column pressure, and are therefore not attractive for chromatographic uses.

U.S. Patent No. 4,389,385 teaches the preparation of porous gels and ceramic materials by dispersing solid particles of an inorganic substance produced by a vapor phase condensation method in a liquid to form a sol. The sol contains colloidal particles which are aggregates of the primary particles. The sol is dried to produce a porous gel of greater than 70% by volume porosity.

### B. Reverse Phase High Pressure Liquid Chromatography

The majority of separations employing high pressure liquid chromatography (HPLC) are performed in the so-called reversed-phase mode. In this mode, the column-packing material is referred to as stationary phase. The most commonly used stationary phases feature a non-polar ligand (e.g., octane or octadecane) covalently-bound to a porous silica particle through a siloxane bond (Si-O-Si) to render the surface hydrophobic. Although these silica-based bonded phases are very useful for a wide range of applications in reversed-phase HPLC, their use is strictly limited to the pH range of between 2 and 8, due to the hydrolytic instability of both the silica support particle and the siloxane bond used to "anchor" the non-polar active group. Thus, the production of a pH-stable reversed-phase support material must involve the development of both a stable, controlled porosity, high surface area support material and a method for rendering the surface permanently hydrophobic.

The eluent, also referred to as the mobile phase, used to elute the various components from the stationary phase is relatively polar, e.g. , an aqueous buffer or a mixture of water and an organic solvent, e.g., aqueous alcohol. Its polarity can be changed by increasing the concentration of the less polar liquid in the mobile phase, a technique known in the art.

Thus relative to the use of ZrO₂-clad silica, a more promising approach to developing a highly stable reversed-phase support, involves replacing the silica with an alternative inorganic material, such as alumina. Although it has been demonstrated that some improvement in pH stability is realized by replacing silica with alumina, the dissolution of alumina in aqueous solutions at extreme pHs (pH<2 and pH>12), even at room temperature, is well known.

As mentioned previously, in addition to the use of a pH-stable support material, the production of a stable, reversed-phase also requires a process for modifying the support material which results in a stable, hydrophobic surface. Silylation is the most widely used method to derivatize silica particles to produce hydrophobic reversed-phase supports. The silylation of inorganic bodies other than silica (e.g., alumina, titania, zirconia, etc.) has been disclosed in U.S. Patent No. 3,956,179. However, it is uncertain whether or not covalent bonds to the support surface are actually formed. In any event, the hydrolytic instability of the siloxane bond is well known, and it is very likely that a Si-O-metal bond will be even more susceptible to aqueous hydrolysis because of the increased polarity of the bond.

An alternate approach to silylation for modifying the surface polarity of inorganic bodies is the sorption of a polymer of desired polarity/functionality onto an SiO₂ or Al₂O₃ support surface followed by crosslinking of the individual polymer chains to one another to impart additional stability to the coating. Reversed-phase supports prepared in this fashion exhibit much improved pH stability compared to those prepared by silylation. It is important to recognize that the formation of a stable, crosslinked polymer layer on the surface of the support does not reduce the need for a stable, inorganic support, since it may not be possible to cover the entire inorganic surface. Although crosslinking of the polymer may keep it in place even as the underlying inorganic support dissolves, dissolution of the support will undoubtedly lead to a reduction in the mechanical stability of the support. In addition, problems related to increasing column back pressure are known to accompany the dissolution of the inorganic support and its subsequent appearance in the mobile phase and transport through the column and the accompanying instrumentation.

Another problem related to the use of silica-based reversed phase supports is the difficulty encountered in the chromatography of amines and other basic solutes. This problem results from the presence of acidic silanol groups (SiOH) on the silica surface. Basic solutes undergo very strong interactions with these silanol groups which may involve cation exchange or hydrogen bonding, depending on the pH of the mobile phase. The problem is exaggerated by the requirement of working in the pH range 2<pH<8 on silica-based columns, since most amines will be protonated in this pH range and protonated amines can readily bond to the silica surface. One obvious approach to improving the chromatography of amines is to work at hydrogen ion concentrations significantly above the ionization constant of the amines so that they are unprotonated. For aliphatic amines, this normally involves working at a pH greater than 11. However, these pH ranges cannot be employed using silica-based columns.

The presence of the aforementioned acidic silanol groups can also lead to irreversible adsorption of many classes of organic molecules onto silica-based reversed-phase supports, a problem which is well known to those versed in the art. This irreversible adsorption is particularly troublesome in the reversed-phase HPLC of proteins. Ultimately, this adsorption will result in a change in the properties of the support and can lead to its destruction.

Reversed-phase HPLC is finding increased use in the area of bioprocessing because of HPLC's great ability to separate and purify materials. At the preparative scale, there are many unique considerations not applicable at the analytical scale. One such consideration is the need to sterilize a chromatography column prior to its use in the purification of a product intended for biological or human use.

### Objects of the Invention

It is, therefore, an object of the present invention to produce support material which is stable over a wide pH range, and which resists dissolution by aqueous media.

Also, it is the object of the present invention to produce a support material which can be regenerated by freeing it from "irreversibly adsorbed" biological or organic residues by treatment at high pH.

It is another object of the present invention to provide a support material for use in biotechnology, that can withstand traditional sterilization techniques involving high pH and heat treatment.

### Summary of the Invention

The present invention provides a support material which comprises porous spherules of zirconium oxide (ZrO₂, 'zirconia'). These spherules display a remarkable physical stability in aqueous media of a pH of 1 to 14. The ZrO₂ spherules are 20-500 µ in diameter, have a surface area of 1-200 m²/g, most preferably 40-150 m²/g; and have pore diameters of from 0.05-50 nm (0.5-500 Å) most preferably 10-30 nm (100-300 Å).

The ZrO₂ spherules of the invention are characterized in that they are prepared by a process consisting essentially of (a) dispersing an aqueous sol containing colloidal ZrO₂ particles in a liquid medium which extracts the water from the dispersed sol to afford gelled ZrO₂ spherules; (b) recovering the gelled spherules from the medium; and (c) heating the gelled spherules to yield solid porous ZrO₂ spherules. This process yields porous particles of ZrO₂ which are essentially spherical. When formed into a bed, the spherules provide improved mobile phase flow characteristics over those exhibited by irregularly-shaped, jagged-edged or angular particles.

In a preferred embodiment of this process, the colloidal ZrO₂ Sol is centrifuged, the supernatant liquid decanted and the residue redispersed in an about equal volume of water. This procedure is preferably repeated a plurality of times (2-5X). The redispersed ZrO₂ yields spherules having a larger pore diameter and an increased pore volume, when they are formed in accord with the present method.

These particulate spherules can be formed into a bed. The bed can also be formed of a mass of spherules which are contained in an immobilized enzyme reactor or other type of bioreactor.

The ZrO₂ spherules of the present invention are employed to immobilize bioactive materials for a variety of purposes, including catalysts, analysis, affinity chromatography and synthetic transformations. Bioactive materials can be strongly sorbed onto the exterior and interior surfaces of the uncoated ZrO₂ spherules, while retaining a large percentage of their initial bioactivity. Useful biomaterials include proteins such as enzymes and antibodies.

In addition, 'irreversibly adsorbed' organic or biological residues can be removed from fouled columns packed with uncoated spherules by flushing the column with a mobile phase at high pH or by injecting pulses of the high pH mobile phase. The term 'irreversible adsorption' refers to the very strong tendency which surface-adsorbed proteins, bio-polymers and the like exhibit to remain sorbed under normal elution conditions, until the mobile phase conditions are changed sufficiently to desorb them.

Therefore, uncoated ZrO₂ spherules are prepared which comprise a biologically active material such as an enzyme or a protein such as an immunoglobulin. Upon depletion of the biological activity, the enzyme or other protein can be removed from the spherules by exposing them to an aqueous medium at high pH, e.g. by washing them with a solution of an alkali metal hydroxide. The spherules, stripped of the biological materials, can then be treated with a buffer to return them to a physiological pH, and subsequently reloaded with the same, or a different bioactive material.

The ZrO₂ spherules may also be exposed in situ to traditional sterilization conditions, for example, by exposing the packing or the packed column to heat and high pH, without significant degradation.

### Detailed Description of the Invention

### I. Zirconium Oxide

In the practice of this invention, a portion, or preferably a majority of the initial zirconium oxide (ZrO₂) used to form the present spherules is in the sol state; a colloidal dispersion of ZrO₂ particles. Such a state is clearly a different physical state than is achieved by the simple dispersion of a silica aerogel in water. The latter easily sediments or precipitates because of its relatively larger particle size and lack of stabilizing counter ions in solution. This is in contrast with a colloidal Sol where the inorganic particles in an aqueous solution usually are not visible to the naked eye.

Sol particles are submicron in particle size and hence will pass through most common filter papers. Sol particles in water do not aggregate because of a stabilizing electrical charge on the surface which is termed a zeta potential. Once the water is removed, the sol particles interact strongly with one another through hydrogen bonding and Van der Waals forces, to provide aggregated Sol particles.

Colloidal dispersions of zirconium oxide suitable for use as the ZrO₂ source to prepare the present spherules are commercially available, e.g., as the Nyacol™ series, Nyacol, Inc., Ashland, MA. These dispersions contain about 20 wt-% ZrO₂, wherein the ZrO₂ particles vary in average diameter, e.g., from about 10-250 nm. For example, Nyacol™ Zr 95/20 is an aqueous dispersion containing 20 wt-% ZrO₂ of colloidal ZrO₂ particles, the majority of which are about 95 nm in diameter.

Non-colloidal ZrO₂ sources may be included along with the colloidal ZrO₂ particles used to prepare these spherules. Thus, chloride, nitrate, sulphate, acetate, formate or other inorganic or organic salts of zirconium such as the oxysalts and alkoxides may be included with the ZrO₂ sol and the mixture used to make spherules. In preferred mixtures, colloidal ZrO₂ particles make up the bulk of the total ZrO₂ present.

Organic compounds may also be included with the ZrO₂ precursors used to prepare the spherules. These organic materials are fugitives which are removed during the firing of the spherules. In particular, water-soluble polymers such as polyvinylpyrolidone, polyethylene glycol, polyethylene oxide, and the like, or latex particles may be included in the liquid mixture used to prepare the spherules. These fugitives may be added to alter the rheology of the precursor solution or the pore structure of the resulting fired spherule.

It is also within the scope of the present invention to include precursors for other metal oxides with the ZrO₂ precursors so as to stabilize a particular crystalline phase of ZrO₂ or to retard grain growth in the fired spherules. Thus, salts or sols of metals such as yttrium, magnesium, calcium, cerium, aluminum, and the like may be included in levels of from approximately 0-20 mole-%. ZrO₂ spherules fired in air or in oxidizing atmospheres which do not contain other oxide additives display either monoclinic, tetragonal or pseudovibic crystal structures when cooled to room temperature. Higher firing temperatures and longer firing times favor the presence of the monoclinic phase. The inclusion of other metal oxides allows the preparation of spherules which possess either monoclinic, tetragonal, or cubic crystalline structures.

Those features of ZrO₂ are well known in the art and are discussed in, for example, An Introduction to Zirconia, Magnesium Elektron Ltd., Twickenham, England, (2d ed., Magnesium Elektron Publication No. 113, July 1986).

### II. Preparation of ZrO₂ Spherules

To prepare the spherical ZrO₂ particles, or 'spherules' of the present invention an aqueous sol containing a colloidal dispersion of ZrO₂ particles is dispersed in a medium which can extract water from the dispersed sol in the form of droplets. Removal of all or a portion of the water results in gelled solid spherules which consist of aggregated sol particles. One medium which may be used is 2-ethyl-1-hexanol as disclosed in US Patent No. 4,138,336. A preferred medium for safety reasons and ease of processing is peanut oil, which is preferably used at a temperature of 80-100°C. The most preferred medium is a mixture of peanut oil and oleyl alcohol which are combined in a ratio of 1:1, and used at a temperature of 80-100°C. Oleyl alcohol possesses a higher extraction capacity than peanut oil and mixtures of the two allow the extraction capacity of the medium to be controlled. Depending upon the ratio of sol to forming medium extraction times of from 1-60 minutes can be used to fully gel the ZrO₂ particles. The gelled spherules may be conveniently separated from the extracting medium, e.g. by filtration.

Once the ZrO₂ particles are condensed into spherules by the above process, thermal treatment at firing temperatures of from 100-1500°C preferably 400-800°C is performed. The resulting fired spherules may be from 20-500 µ is in diameter and can possess a surface area of 1-200 m²/g and pore diameters of from 0.05-50 nm (0.5-500 Å). These particles have high mechanical strength and exceptional stability to aqueous solutions of pHs of 1-14.

A column 'fouled' by repeated injections of large amounts of material to the point that a marked change in characteristics is observed, can be stripped of irreversibly adsorped material. The original column performance can be restored by pulsing the column with 100 µl injection of 1 M NaOH or by flushing the column for about 0.5-10 hrs with aqueous alkali metal hydroxide i.e. with a 0.1 M NaOH solution.

The stability of the uncoated ZrO₂ spherules to sterilizing conditions can be demonstrated by heating a previously characterized column to 100°C while pumping a 1 M NaOH solution through it for 12-24 hrs. Recharacterization of the column demonstrates that no significant change in column properties or decreased retention of a non-polar substance has taken place.

### III. Bioactive Materials

A wide variety of bioactive materials can be bound to the uncoated spherules by presently-available techniques so that their bioactivity is retained and prolonged or 'stabilized' with respect to the unbound bioactive material. For example antibodies or enzymes can be bound to the uncoated spherules in high concentrations by agitating an aqueous mixture of degassed spherules and antibody in a buffer, e.g. for 0.1-5 hrs under ambient conditions. For a review of other noncovalent and covalent enzyme-binding methodologies, see R.A. Messing (US Patent No. 3,850,751).

Enzymes capable of being bound and stabilized as described herein include a wide variety of enzymes which may be classified under six general groups: hydrolytic enzymes, redox enzymes, transferase enzymes, lyases, isomerases and ligases. The first group, hydrolase enzymes include proteolytic enzymes which hydrolyze proteins, e.g., papain, ficin, pepsin, trypsin, chymotrypsin, bromelin, keratinase; carbohydrases which hydrolyze carbohydrates, e.g., cellulase, glucuronidase, amylase, maltase, pectinase, chitinase; esterases which hydrolyze esters; e.g., lipase, cholinesterase, lecithinase, phosphatase; nucleases which hydrolyze nucleic acid, e.g., ribonuclease, deoxyribonuclease; and amidases which hydrolyze amines, e.g., arginase, aspariginase, glutaminase, and urease. The second group are redox enzymes that catalyze oxidation or reduction reactions. These include glucose oxidase, catalase, peroxidase, lipoxidase, and cytochromes. The third group are transferase enzymes that transfer groups from one molecule to another. Examples of these are glutamic-pyruvic transminase, glutamic-oxalacetic transaminase, transmethylase, phosphopyruvic transphosphorylase and dehydrogenase. The fourth group are lyase enzymes that catalyze the cleavage of C-C, C-O, C-N and other bonds by elimination, leaving double bonds, or conversely, adding groups to double bonds. Examples of these are pyruvate decarboxylase, amino acid decarboxylases, aldolase, fumarate hydratases, aconitate hydratases and ammonia lyase. The fifth group are isomerase enzymes that catalyze the dehydrogenation and epimerization of amino acids and sugars. An example of an isomerase is phosphoglucomutase. The sixth group are ligase enzymes that catalyze the synthetic linking of two molecules, simultaneously with the breakdown of ATP. Examples of these are aminoacyl-tRNA synthetases and biotinyl-dependent carboxylases.

Other proteins capable of being bound and stabilized as described herein include Con-A, Protein-A, plasma immunoglobulins, monoclonal antibodies, bioactive polypeptides such as serum proteins and immunomodulators, e.g., lymphokines.
Other examples of proteins which are bound by the present spherules as provided in the working example, hereinbelow.

### IV. Modification with Organophosphorus Compounds

For some applications, it is desirable to further deactivate or modify the surface of the uncoated ZrO₂ spherules. This can be used to enhance the ability of the spherules to separate polar compounds such as amines and carboxylic acids, or to modify retention. This deactivation can be accomplished by treating the uncoated ZrO₂ spherules with an organophosphorus compound in a suitable solvent for the organophosphorus compound. Useful organophosphorus compounds include allylphosphonate, octyl phosphonate, phenyl phosphonic acid, napthyl phosphonic acid, phenyl phosphinic acid, phenylphosphoric acid and diallyl phosphorate. Preferred organophosphorus compounds include the unsaturated organophosphonic acids and the water-soluble salts thereof.

Useful solvents for the organophosphorus compound include aqueous alcohol, e.g., a solution of water and a (C₁-C₅)alkanol. The ZrO₂ spherules are preferably coated by agitating the spherules in a solution of the organophosphorus compound so that the weight ratio of the organophosphorus compound to spherules is about 0.25-1:1. The treated particles are then separated from the treating solution and dried.

The invention will be further described by reference to the following detailed examples.

### Preparation Example 1

Peanut oil (3 liters) was placed in a 4 liter beaker and heated to 90°C. A mechanical agitator was inserted and the peanut oil was vigorously stirred. One hundred grams of Nyacol™ Zr 95/20, a colloidal ZrO₂ manufactured by Nyacol, Inc. and containing 20 wt-% of ZrO₂, primarily as about 95 nm particles, was sprayed into the peanut oil through an aerosol atomizer. After approximately 30 minutes, the batch was filtered through a No. 54 Whatman filter. Approximately 17 g of solids were recovered, which were predominately spherules having a diameter of < 30 µ.

### Preparation Example 2

Peanut oil (600 g) and 600 g of oleyl alcohol were mixed and heated to about 90°C. Under vigorous agitation, 100 g of Nyacol™ Zr 95/20 was sprayed into the peanut oil/oleyl alcohol mixture as described in Example 1.

After 30 minutes, the batch was filtered and the particles collected. The particles were predominantly (ca:70%) spherules having a diameter of < 50 µ.

Spherules prepared as described in Preparation Examples 1 and 2 were thermally treated at a series of temperatures and the surface area, average pore diameter and pore volume were measured by nitrogen adsorption isotherm on a Quantasorb surface area analyzer. These results are summarized in Table I, below.

**Table I**

| Firing Temp (°C)* | Surface Area (m²/g) | Average Pore Diameter | | Pore Volume (%) |
|---|---|---|---|---|
| | | nm | Å | |
| 400 | 142 | 4.2 | 42 | 47 |
| 500 | 92 | 7.1 | 71 | 50 |
| 600 | 34 | 11.0 | 110 | 36 |
| 800 | 17 | 20.5 | 205 | 34 |
| 900 | 14 | 22.0 | 220 | 31 |

| | | | | |
|---|---|---|---|---|
| * 6 hrs | | | | |

The data summarized on Table I show that it is possible to increase the average pore diameter by increasing the firing temperature from 400 to 900°C. The surface area and pore volume decrease with increasing firing temperature. Chromatographic activity of the ZrO₂ spherules is determined by the parameters of surface area, average pore diameter and pore volume. Accordingly, the appropriate firing temperature is selected.

### Preparation Example 3

The procedure of Preparation Example 2 was used to prepare spherules using Nyacol™ Zr 50/20, a colloidal ZrO₂ supplied by Nyacol Inc. (50 nm ZrO₂ colloid size) as the ZrO₂ source.

### Preparation Example 4

The procedure of Preparation Example 2 was used to prepare spherules using Nyacol™ Zr 150/20, a colloidal ZrO₂ supplied by Nyacol Inc, (150 nm ZrO₂ colloid size) as the ZrO₂ source.

Table II summarizes the surface area, average pore diameter and pore volume of spherules prepared as per Preparation Examples 2-4 and fired at 600°C for 6 hrs.

**Table II**

| ZrO₂ Source* | ZrO₂ Colloid size (nm) | Surface Area (m²/g) | Average Pore Diameter | | Pore Volume (%) |
|---|---|---|---|---|---|
| | | | nm | Å | |
| Zr 50/20 | 50 | 33 | 9.2 | 92 | 31 |
| Zr 95/20 | 95 | 34 | 11.0 | 110 | 36 |
| Zr 150/20 | 150 | 40 | 14.7 | 147 | 45 |

| | | | | | |
|---|---|---|---|---|---|
| * Nyacol™ series | | | | | |

The data summarized in Table II show that it is possible to control the average pore diameter of the fired spherules by appropriate selection of the colloid size of the ZrO₂ source. Larger colloids produce fired spherules with larger pore diameters and pore volumes.

### Preparation Example 5

Nyacol™ Zr 95/20 colloidal ZrO₂ was placed in a laboratory centrifuge and sedimented. The supernatant was decanted and discarded. The sedimented ZrO₂ was redispersed in an equal volume of distilled water. Spherules were prepared from this centrifuged sol following the procedures of Preparation Example 2.

### Preparation Example 6

The centrifugation procedure of Preparation Example 5 was performed and the redispersed sol was subsequently recentrifuged to sediment, the supernatant decanted off and the ZrO₂ redispersed. Spherules were prepared from this doubly centrifuged sol following the procedure of Preparation Example 2.

### Preparation Example 7

The double centrifugation procedure used in Preparation Example 6 was performed and the redispersed sol was subsequently recentrifuged to sediment, the supernatant decanted, and the ZrO₂ redispersed. Spherules were prepared from this triply centrifuged sol following Preparation Example 2.

Table III summarizes the surface area, pore diameter and pore volume of spherules prepared as per Preparation Examples 2, 5, 6 and 7 and heated to 600°C for 6 hrs.

**Table III**

| ZrO₂ Source* | Surface Area (m²/g) | Average Pore Diameter | | Pore Volume (%) |
|---|---|---|---|---|
| | | nm | Å | |
| Zr 95/20 | 34 | 11.0 | 110 | 36 |
| Zr 95/20 cent.(1x) | 50 | 16.2 | 162 | 55 |
| Zr 95/20 cent.(2x) | 52 | 23.5 | 235 | 62 |
| Zr 95/20 cent.(3x) | 46 | 25.0 | 250 | 62 |

| | | | | |
|---|---|---|---|---|
| * Nyacol™ Zr series | | | | |

Centrifugation, removal of the supernatant, and redispersion of the colloidal ZrO₂ starring material results in increases in the average pore diameter, pore volume and surface area of fired spherules. This increase is believed to result from the removal of small (ca. 5-10 nm) colloidal ZrO₂ particles which are known to be present in the Nyacol™ Zr series sols as a minor component. Many of these smaller ZrO₂ particles remain suspended during centrifugation and are removed when the supernatant is discarded prior to redispersion of the larger sedimented ZrO₂ particles. If present, these small ZrO₂ particles are believed to increase the packing density of the spherules by filling the interstices between larger ZrO₂ particles and therefore decreasing the average pore diameter, pore volume and surface area of the fired spherules.

It is also possible that sedimentation by centrifugation may result in agglomeration of the colloidal ZrO₂ particles into aggregates which pack together in a more open structure (effectively behaving as larger particles) than unaggregated particles.

Regardless of mechanism, the centrifugation treatments described in Preparation Examples 5-7 provide a method of preparing spherules with increased average pore diameter, pore volume and surface area relative to spherules prepared from untreated colloidal ZrO₂ sols.

The following examples demonstrate the use of the ZrO₂ spherules to immobilize proteins.

### Example 8

ZrO₂ spherules with a diameter of approximately 30 µ is and a surface area of 50 m²/g and an average pore diameter of 12.4 nm (124 Å) were used. Mouse antihuman IgE antibody was purified and radioinitiated (I¹²⁵) by the method of S. M. Burchiel et al. J. Immunol. Meth., 69, 33 (1984); K. L. Holmes et al. PNAS USA, 82, 7706 (1985), and diluted with unlabelled antibody to yield a specific radioactivity of 5,000 cpm/µg. A portion of 250 µl of antibody (250 µg/ml in 5 mM Tris, pH 8.0) was added to tubes containing 10 mg of spherules. The mixture was briefly evacuated, then rocked at ambient temperature for the appropriate time, 5-120 min., with three replicates for each time point. The tubes were centrifuged and rinsed twice with 1 ml of buffer. The spherules were transferred to a fresh tube along with 2 ml of buffer, the buffer removed and the radioactivity of each tube was determined in a Packard Model 5230 gamma scintillation counter. The amount of bound protein in ng, converted from cpm, is shown in Table V.

**Table V**

| Time (Min) | Antibody Bound/mg Spherules |
|---|---|
| 10 | 54 ng |
| 20 | 66 ng |
| 30 | 72 ng |
| 60 | 69 ng |
| 120 | 62 ng |

### Example 9

Using the same materials and techniques described in Example 8 the extent of antibody in 2 hr incubations as a function of its concentration (1-250 µg/ml) was determined. The averages of three replicates show a saturation (Table VI). Double-reciprocal analysis of these data extrapolate to 100 µg antibody bound per g spherule at saturation.

**Table VI**

| Conc. Protein (µg/ml) | Antibody Bound/mg Spherules |
|---|---|
| 1 | 1.5 ng |
| 5 | 7.5 ng |
| 10 | 14.0 ng |
| 50 | 38.0 ng |
| 250 | 62.0 ng |

### Example 10

### A. Trypsin Immobilization

Solutions (2 mg/ml) of trypsin, a 24 KDa proteolytic enzyme and bovine serum albumin (BSa), a 67 KDa protein, were bound to 70 mg of the ZrO₂ spherules (average pore diameter 10nm (100 Å) surface area of 30 m²/g) in 5 mM tris, pH 8.0 by agitating the degassed spherules in 1.0 ml of buffer for 17.5 hr. Trypsin (15.3 mg) and <0.2 mg (BSA) bound per g of spherule, a proportion which might be expected from their relative sizes and the size of the pores.

Trypsin was assayed using the thioesterase assay disclosed by P. L. Coleman et al., Meth. Enzymol., 80, 408 (1981). The bound spherules were suspended in 1 ml of buffer and a 5 µl aliquot was added to a tube containing 1.0 ml of substrate. After 2.5 min of continuous shaking, a citrate-soybean trypsin inhibitor (STI) Solution was added to quench the reaction. It was rapidly centrifuged and the supernatant removed for determination of the absorbance (A) at 412 nm. Assays were performed on trypsin spherules, BSA spherules and the supernatant from trypsin spherules. The assay was also performed with the trypsin inhibitor in the substrate solution to determine whether it was able to inhibit the bound trypsin. The results of these assays are summarized on Table VII, below.

**Table VII**

| Sample | Trypsin Activity (A at 412 nm) | |
|---|---|---|
| | -STI | +STI |
| Trypsin spherules | 2.36 | 1.79 |
| BSA spherules | 0.10 | 0.13 |
| Trypsin supernatant | 0.19 | 0.12 |

The results (Table VII) indicate that about 75% of the bound activity is unavailable to STI, even though STI is smaller than trypsin. In addition, only 4% of the activity is attributable to unbound trypsin, a surprisingly low value given the inefficient batch washing method which was used.

Calculations based on these observations demonstrated several unexpected results. For example, 15 mg of trypsin/g ZrO₂ corresponds to 51 mg/ml using 3.3 g/ml as the density of the spherules. This corresponds to a trypsin concentration of 2 nm in the column. A check on this may by made by estimating the expected A at 412 nm for the assay. In these assays, the spherule-bound enzyme was 0.21 µM, the kcat for the substrate is 75/sec [G. D. J. Green et al., Anal. Biochem., 93, 223 (1979)] and the extinction coefficient is 14,100, yielding an estimated 3.3 absorbance change, which compares favorably with the 2.4 observed. Since chromogen was present in amount sufficient to give only 2.8 A at 412 nm, it is safe to assume that nearly all of the bound trypsin is active. Thus, an extraordinary amount of protein is bound and retains its enzymic activity.

### B. Chymotrypsinogen-Chymopapain-BSA Immobilization.

The procedure of Example 7 was employed to prepare ZrO₂ spherules having 240 Å pores and a surface area of 27 m²/g. Small columns were poured, each containing about 1.0 g of spherules, and were equilibrated with either 20 mM tris-chloride buffer (pH 8.0) or 50 mM sodium acetate buffer (pH 4.5). Chymotrypsinogen (24.5 kDa) and chymopapain (32 kDa) were dissolved in the tris buffer and BSA was dissolved in the acetate buffer. Protein-containing solution was continuously added to the column until the 280 nm absorbance of the eluate equalled that of the starting solution. Unbound protein was rinsed from the column, and the amount of bound protein was calculated from the difference between that added and that recovered in the eluate.

Chymotrypsinogen and chymopapain bound at 76.9 mg and 24.5 mg of protein/g of spherules at pH 8.0, respectively and 64 mg of BSA bound per gram of spherules at pH 7.5. Converting these values into binding densities per ml of column volume yields 254, 81 and 211 mg/ml of protein, respectively.

The fact that at acidic pH, albumin binds to a greater extent than does the smaller chymopapain, and almost to the extent as the even smaller chymotrypsinogen suggests that the latter enzymes would bind to even greater densities at lower pH, i.e., below their pla.

### Preparation Example 11

### Polymer Adsorption/Crosslinking.

Preparation A - Heavily loaded ZrO₂: A solution of 0.55 g of polybutadiene (Aldrich Chemical Co., Milwaukee, WI, m.w. 4500, Cat. No. 20-050-6) in 50 ml of pentane was added to 3.5 g of ZrO₂ spherules prepared as described in Example 2 (fired at 600°C for 6 hrs; particle size = 20-45 microns] which had been boiled in CO₂-free water to fully hydrate the surface and then dried at 125°C. The slurry was placed in an ultrasonic bath and a water aspirator vacuum applied. Dicumyl peroxide (DCP) (0.01 g) was then added and the slurry was again placed in an ultrasonic bath and a vacuum applied. The pentane was removed in vacuo and the material dried at 70°C under vacuum. The material was then heated in a tube furnace at 200°C for 2 hrs and then washed successively with pentane, toluene, methylene chloride, tetrahydrofuran, methanol and 0.1 M sodium hydroxide. Elemental analysis of the coated spherules showed a carbon load of 7.7%. A duplicate sample was prepared in an identical fashion and had a carbon load of 7.5%. Because of the extremely heavy load of polybutadiene, the specific surface area of the porous spherules, as determined by a BET measurement, decreased from 50.4 to 4 m²/gm.

Preparation B - Lightly loaded ZrO₂: 35 ml of a solution of 0.09 g of polybutadiene in pentane was added to 3.5 g of ZrO₂ spherules and the resultant slurry was placed in an ultrasonic bath and a water aspirator vacuum applied. Pentane (10 ml) containing 0.002 g of DCP was then added and the slurry was again placed in an ultrasonic bath and a vacuum applied. The slurry was then shaken for one hr and the supernatant removed by filtration. The material was then washed as described in Preparation A. Elemental analysis of the coated spherules showed 0.84% carbon, while the BET results showed a specific surface area of 38.7 m²/gm. The decrease in specific surface area from 50.4 to 38.7 m²/gm is similar to the reduction in surface area which occurs upon silylation of typical inorganic supports.

Preparation C - Intermediate load: A solution of 0.27 g of PBD in 50 ml pentane was added to 3.0 g of ZrO₂ spherules (mean particle diameter 3.5 microns). The slurry was placed in an ultrasonic bath and a vacuum applied. 5.2 mg of DCP in 10 ml of pentane were then added. The methodology of Preparation A was then followed. Elemental analysis showed 2.7% carbon.

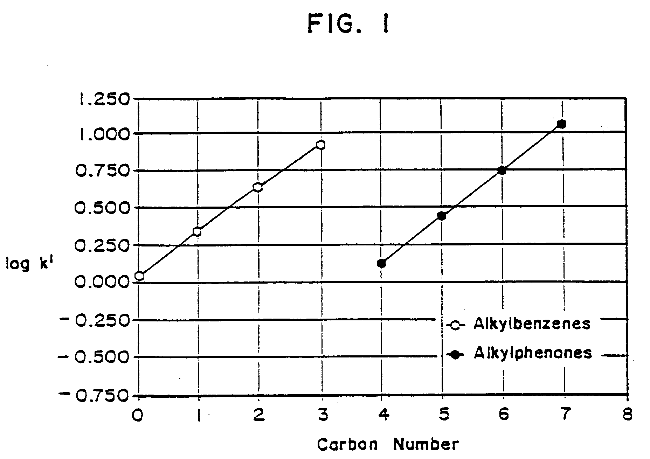

It is clear from the results of carbon analysis that carbon had been deposited on the surface of the ZrO₂ spherules. Figure 1 further demonstrates the reversed-phase nature of the polymer-modified ZrO₂ spherules as exhibited by a 5 cm x 0.46 cm column packed using Preparation C. The linearity of the log k' (capacity factor) vs. carbon number plot for the members of a homologous series of alkylphenones is clearly indicative of a reversed-phase retention mechanism.

### Example 12

ZrO₂ spherules prepared by the procedure of Preparation Example 2 (3.4g surface area: 60 m²/g; pore diameter: 95 Å) were treated with a solution of 1.6 g of allylphosphonate in 50 ml of 95/5 (v/v) methonal/water. After 'ultrasonicating' under vacuum and shaking for one hr. the supernatant was removed by filtration and the phosphonate-treated ZrO₂ was dried at 70°C for 12 hrs. The material was then modified with PBD according to Preparation Example 11, Preparation C. In this manner the residual ZrO₂ surface was deactivated as is clearly shown by the data in Table IX. Note that carboxylic acids are not eluted on the non-phosphonated ZrO₂ material but are eluted on the phosphonated material.

**Table IX**

| Solute | k'(untreated) | k'(treated) |
|---|---|---|
| Toluene | 0.46 | 0.49 |
| Benzoic Acid | not eluted | 6.1 |

### Example 13

Several 100 µl injections of cytochrome C were made on a column packed with material prepared as described in Preparation Example 11, Preparation C. The retention of cytochrome C on this material decreased due to 'irreversible' adsorption of protein upon each injection.

The column was then 'pulsed' with 5, 100 µl injections of 1 M NaOM in order to strip the 'irreversibly adsorbed' cytochrome C. The effect of the puses is to strip the column of adsorbed protein such that the original retention characteristics can be regenerated.

### Example 14

The ability of the subject matter to withstand sterilizing conditions was demonstrated by taking a material prepared as described in Preparation Example 11, Preparation C, and evaluating its chromotographic characteristics, by exposing it to 1 M NaOM at 100°C for 1 hr and then reevaluating the chromotographic properties. As shown by the data in Table X, there was no decrease in retention of nonpolar substances upon challenging the packing in this fashion.

**Table X**

| Solute | k' | |
|---|---|---|
| | Before Treatment | After Treatment |
| Benzene | 1.36 | 1.47 |
| Toluene | 2.68 | 3.01 |
| Ethyl Benzene | 4.83 | 5.57 |
| Propyl Benzene | 9.21 | 10.86 |

A second column (ES Industries, Marlton, NJ) which uses an alumina support modified by the method of G. Shomberg, LC-GC, 6, 36 (1988), was challenged with a mobile phase of 1M NaOH, which was collected in two fractions. The first corresponded to an elution time of 1 hr and the second to an additional elution of 2.25 hrs.

The elements were analyzed via an inductively coupled plasma spectrometer. The concentration of aluminum- in the eluent from the second column corresponded to the dissolution of a total of 10% of the mass of the alumina in the column.

In marked contrast, zirconium was absent at a level of detectability of 0.03 µg/ml. Even if Zr was present at the detection limit, this would correspond to loss of less than 0.001% of the mass of ZrO₂ on the test column.

The invention has been described with reference to various specific and preferred embodiments and techniques. However it should be understood that many variations and modifications may be made while remaining within the scope of the invention.

## Claims

1. Porous ZrO₂ spherules of 20-500 µm (microns) in diameter, 1-200 m²/g surface area and of 0.05-50 nm (0.5-500 Å) pore diameter; wherein said spherules are substantially stable in an aqueous solution of a pH of 1-14, and which comprise an immobilized protein.

2. The ZrO₂ spherules of claim 1 which comprise an immobilized enzyme.

3. The ZrO₂ spherules of claim 1 which comprise an immobilized antibody.

4. The ZrO₂ spherules of claim 1 which comprise a monoclonal antibody.

5. The ZrO₂ spherules of claim 1 which comprise an immobilized serum immunoglobulin.

6. The ZrO₂ spherules of claim 1 which comprise an immobilized immunomodulator.

7. The ZrO₂ spherules of claim 6 which comprise an immobilized lymphokine.
